# EUROPEAN PATENT APPLICATION

(11) **EP 4 617 260 A1**
(43) Date of publication of application: **17.09.2025**
(21) Application number: 22965116.1
(22) Date of filing: 09.11.2022
(51) Int. Cl.: C07D 231/20

(54) **PRODUCTION METHOD FOR 1-ALKYL-5-HYDROXYPYRAZOLE**

(71) Applicant: Mitsubishi Gas Chemical Next Company, Inc., Minato-ku Tokyo 105-7116 (JP)
(72) Inventor: KONISHI, Ryota, Niigata-shi, Niigata 950-3126 (JP); KAMIMURA, Sadao, Niigata-shi, Niigata 950-3126 (JP); YAMADA, Yu, Tokyo 105-7116 (JP)
(74) Representative: Gille Hrabal Partnerschaftsgesellschaft mbB Patentanwälte
(86) International application number: PCT/JP2022/041682
(87) International publication number: WO 2024/100791

(57) **Abstract**

The present invention provides a production method that can easily produce a 1-alkyl-5-hydroxypyrazole by subjecting a dialkylaminoacrylate ester and an alkylhydrazine to a reaction as represented by the following reaction formula A. (Reaction formula A) (In the formula, R₁, R₂, and R₃ are each independently a C1 to C6 alkyl group, R₄ is a hydrogen atom, a C1 to C6 alkyl group, a C2 to C6 alkenyl group, a C3 to C6 cycloalkyl group, or an aryl group, which groups are optionally substituted with halogen, a hydroxy group, an alkoxy group, or a C1 to C6 alkyl group.)

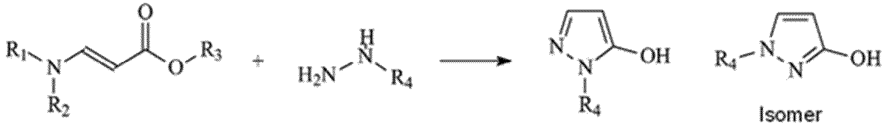

## Description

### Technical Field

The present invention relates to a method for producing a hydroxypyrazole, and, in particular, to a novel method for producing a 1-alkyl-5-hydroxypyrazole.

### Background Art

1-Alkyl-5-hydroxypyrazoles are used as pharmaceuticals and an intermediate for producing crop protection agents typically including herbicides. For example, a 1-alkyl-5-hydroxypyrazole is disclosed, in pharmaceuticals, in an anti-inflammatory agent based on the VR1 receptor antagonism, an antiasthmatic drug based on the 5-LO blocking effect, and a mental disorder remedy, and in agrochemicals, as an intermediate of multiple herbicides for a farm. Thus, a method for producing a 5-hydroxypyrazole, which is considered as a useful compound, attracts attention. The following methods have heretofore been known as main methods for producing a hydroxypyrazole.
1) A production method in which a dialkyl alkoxymethylenemalonate and an alkylhydrazine are subjected to cyclization to synthesize an alkyl 1-alkyl-5-hydroxypyrazole-4-carboxylate, and then, the reaction product is subjected to simultaneous hydrolysis and decarboxylation, thereby producing a 1-alkyl-5-hydroxypyrazole (PTL 1)
2) A production method in which hydrazine is added to an acrylate ester to form a 3-hydrazinopropionate ester, which is then subjected to a dehydration condensation reaction with an aldehyde to produce a corresponding hydrazone, which is then subjected to cyclization, thereby producing a 1-alkyl-5-hydroxypyrazole (PTL 2)
3) A production method in which a 3-alkoxyacrylate ester is reacted with an alkylhydrazine to directly produce a 1-alkyl-5-hydroxypyrazole (PTLs 3 and 4)

### Citation List

### Patent Literature

PTL 1:JPS 61-257974 A
PTL 2:JPH 07-30031 B
PTL 3:US 6,392,058
PTL 4:WO 2017/004674

### Summary of Invention

### Technical Problem

The production method of the above 1) needs a lot of steps. A dialkyl alkoxymethylenemalonate which is a raw material is obtained, which is then cyclized with an alkylhydrazine to synthesize an alkyl 1-alkyl-5-hydroxypyrazole-4-carboxylate, followed by further hydrolysis reaction and decarboxylation reaction, whereby the target 1-alkyl-5-hydroxypyrazole can be obtained at last. Furthermore, the obtained reaction product contains, in addition to the 1-alkyl-5-hydroxypyrazole, a 1-alkyl-3-hydroxypyrazole which is a positional isomer simultaneously produced, and a complicated procedure is needed for separating the isomer from the target compound. Thus, this synthetic method gives a low yield.

The production method of the above 2) is also complex due to a lot of steps needed. A 3-hydrazinopropionate ester which is formed by an addition reaction of hydrazine to an acrylate ester is subjected to a dehydration condensation reaction with an aldehyde to produce a corresponding hydrazone, followed by cyclization, whereby a 1-alkyl-5-hydroxypyrazole can be obtained at last. This complicated method leads to production of a lot of byproducts, resulting in a low yield.

In the production method of the above 3), a 1-alkyl-5-hydroxypyrazole can be obtained in one step by using a 3-alkoxyacrylate ester, but the 3-alkoxyacrylate ester is difficult to produce and is expensive.

Here, for producing the 3-alkoxyacrylate ester which is used as a raw material in the production method of the above 3), the following methods are known.
(1) A reaction between methanol and a propiolate ester which is expensive; (2) a reaction between α,α-dichlorodiethyl ether which is expensive and is difficult to synthesize and a bromoacetate ester which is also expensive; (3) a reaction between a bromoacetate ester which is expensive and a trialkyl formate; (4) elimination of methanol from a 3,3-dialkoxypropionate ester obtained by a reaction between methyl propiolate which is expensive and methanol; (5) a reaction between a 3-(N-acetyl-N-alkyl)amino-3-methoxypropionate ester and methanol; (6) a reaction of an acrylate ester with an alkylamine and acetic anhydride; (7) a reaction between a ketene which is difficult to handle and a trialkyl orthoformate; (8) a reaction between an acrylate ester and methanol catalyzed by palladium and copper; (9) a reaction between trichloroacetyl chloride and a vinyl alkyl ether; (10) a reaction between α,α,α-trichloro-β-methoxybuten-2-one and methanol; and (11) a reaction between a 3-hydroxyacrylate ester sodium salt and an alcohol.

Thus, a 3-alkoxyacrylate ester cannot be easily produced, and hence, may be expensive. Accordingly, the production method of the above 3) is a noneconomic method.

As a result, the synthetic routes are not satisfactory as an economic and efficient production method of a 1-alkyl-5-hydroxypyrazole.

The present invention is to solve such a problem involved in the conventional arts as described above. The problem that the present invention is to solve is to provide an industrial method for producing a 1-alkyl-5-hydroxypyrazole in a simpler manner and in a higher yield and higher purity with more available raw materials as compared with conventional methods.

### Solution to Problem

The present inventors have found that, by focusing on a dialkylaminoacrylate ester as a raw material that is easily reacted and is easily available and subjecting such a dialkylaminoacrylate ester and an alkylhydrazine to a reaction as shown in the following reaction formula A, a 1-alkyl-5-hydroxypyrazole can be obtained in a simple manner, thus completing the present invention. According to this reaction, a high purity 1-alkyl-5-hydroxypyrazole can be obtained in a high yield with a small amount of a positional isomer produced as a byproduct.

### (Reaction formula A)

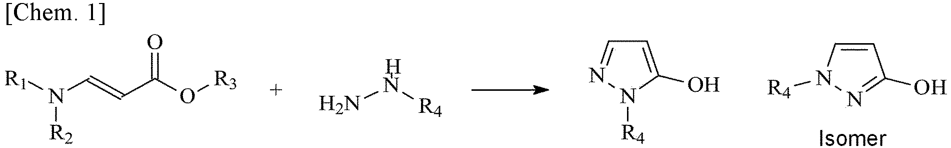

(In the formula, R₁, R₂, and R₃ are each independently a C1 to C6 alkyl group, R₄ is a hydrogen atom, a C1 to C6 alkyl group, a C2 to C6 alkenyl group, a C3 to C6 cycloalkyl group, or an aryl group, which groups are optionally substituted with halogen, a hydroxy group, an alkoxy group, or a C1 to C6 alkyl group.)

### Advantageous Effects of Invention

According the method for producing a 1-alkyl-5-hydroxypyrazole according to the present invention, it is possible to obtain a 1-alkyl-5-hydroxypyrazole which is useful in production of an agricultural herbicide and the like from an easily available dialkylaminoacrylate ester by a simple operation with a short process, in a simpler manner with a higher yield and a higher purity as compared with conventional methods, and thus, a production method that can be used as an industrial production method is provided.

### Description of Embodiments

In the method for producing a 1-alkyl-5-hydroxypyrazole according to the present invention, as shown in the Reaction formula A above, a dialkylaminoacrylate ester represented by the general formula (1) and an alkylhydrazine represented by the general formula (2) are reacted to thereby produce a 1-alkyl-5-hydroxypyrazole represented by the general formula (3).

Hereinunder, the method for producing a 1-alkyl-5-hydroxypyrazole according to the present invention will be specifically described with respect to the compounds used as starting materials, the target compound, the production method including the reaction method, and the like.

### (Dialkylaminoacrylate ester)

The dialkylaminoacrylate ester as a starting material is represented by the following general formula (1). General formula (1):

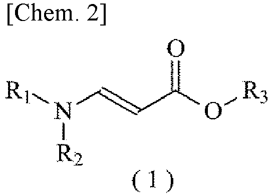

In the formula, R₁, R₂, and R₃ are each independently an alkyl group having 1 to 6 carbon atoms, i.e., a C1 to C6 alkyl group. The alkyl group includes both a linear alkyl group and a branched alkyl group. Among the alkyl groups, as R₁ and R₂, a methyl group and an ethyl group are preferred from the viewpoints of easy removal and economy, and a methyl group is particularly preferred. As R₃, a methyl group and an ethyl group are preferred from the viewpoints of easy removal and economy, and an ethyl group is particularly preferred.

The dialkylaminoacrylate ester represented by the formula (1) has been increasingly demanded as a raw material of fluoroquinolone synthetic antibacterial drugs which have recently been significantly increasingly demanded, such as Ciproxan, Tarivid, Cravit, and Norfloxacin.

Such a dialkylaminoacrylate ester represented by the formula (1) can be easily synthesized by reacting carbon monoxide or a formate ester with an acetic acid ester and a sodium alkoxide, followed by reacting therewith a dialkylamine or a mineral acid salt thereof. For example, such a dialkylaminoacrylate ester is also available as a commercial product from Tokyo Chemical Industry Co., Ltd. or FUJIFILM Wako Pure Chemical Corporation, for example.

### (Alkylhydrazine)

The alkylhydrazine which is reacted with the dialkylaminoacrylate ester is a compound represented by the general formula (2).

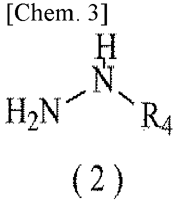

In the formula, R₄ is a hydrogen atom, an alkyl group having 1 to 6 carbon atoms, i.e., a C1 to C6 alkyl group, a C2 to C6 alkenyl group, a C3 to C6 cycloalkyl group, or an aryl group, which groups are optionally substituted with halogen, a hydroxy group, an alkoxy group, or a C1 to C6 alkyl group.

Here, the present invention has a main object to produce a 1-alkyl-5-hydroxypyrazole, and the case of a 1-alkyl-5-hydroxypyrazole is described. In the case where R₄ is a hydrogen atom, an alkenyl group, or an aryl group, the products are respectively 5-hydroxypyrazole, a 1-alkenyl-5-hydroxypyrazole, and a 1-aryl-5-hydroxypyrazole, and has a substituent different from an alkyl group. However, also in the cases, such a produce can be synthesized in the same manner as in the synthesis of a 1-alkyl-5-hydroxypyrazole using an alkylhydrazine as described in the present invention.

Accordingly, as a result of allowing hydrazine derivatives used for the reaction to include hydrazines having other substituents than alkyl groups, in the present description, the compound represented by the general formula (2) is collectively referred to as "alkylhydrazine" with the case of other than an alkyl group included therein although such a substituent cannot be strictly said to be "alkyl". In the same manner, the compound represented by the general formula (3) which is a reaction product obtained by using an alkylhydrazine is also collectively referred to as a "1-alkyl-5-hydroxypyrazole" with the case of not being an alkyl group included therein.

The alkyl group having 1 to 6 carbon atoms, i.e., the C1 to C6 alkyl group which is used for the reaction includes both a linear alkyl group and a branched alkyl group. Among the alkyl groups, a methyl group and an ethyl group are preferred from the viewpoint of economy, and a methyl group is particularly preferred.

The alkenyl group having 2 to 6 carbon atoms, i.e., the C2 to C6 alkenyl group may be linear or branched. The position of the double bond may be any position, either terminal or middle. Such an alkenyl group is preferably an allyl group from the viewpoints of bioactivity and applicability of a further modification reaction.

The cycloalkyl group having 3 to 6 carbon atoms, i.e., the C3 to C6 cycloalkyl group includes a cycloalkyl group on which an alkyl group is substituted and cycloalkyl groups bonded via an alkylene group. In the case of a cycloalkyl group, the number of carbon atoms means the number of carbon atoms that constitutes the ring, and the 3 to 6 carbon atoms do not include the carbon atoms of an alkyl group or an alkylene group that is bonded to the ring. The cycloalkyl group is preferably a cyclopropyl group from the viewpoint of bioactivity.

These groups may be substituted with halogen, a hydroxy group, an alkoxy group, or a C1 to C6 alkyl group, but since an increase in the number of carbon atoms of R₄ in the formula leads to a decrease in the reactivity, the upper limit of the number of carbon atoms is preferably C18. Note that, in the case where R₄ is a C1 to C6 alkyl group and a C2 to C6 alkenyl group, even if it is considered that the C1 to C6 alkyl group is substituted on the groups, the number of carbon atoms of the alkyl group and alkynyl group needs to be 6 or less, and thus, it is not necessary to take the case where a C1 to C6 alkyl group is substituted thereon into account.

In use as an intermediate raw material of a medicine, an agricultural chemical, or the like, as the substituent R₄, a substituent that is suited to the intermediate raw material of the medicine, agricultural chemical, or the like is selected, but in general, the substituent R₄ is an alkyl group or an aryl group in many cases.

The alkylhydrazine can be synthesized by alkylation of hydrazine. For example, the alkylhydrazine is also available as a commercial product from Tokyo Chemical Industry Co., Ltd. or FUJIFILM Wako Pure Chemical Corporation, for example. Such an alkylhydrazine is also commercially available in the form of an aqueous solution, and the concentration is different depending on the type of the alkylhydrazine but in general, is approximately 30 to 50%. In the production method of the present invention, the alkylhydrazine can be used as it is or in an aqueous solution as is commercially available.

### (1-Alkyl-5-hydroxypyrazole)

A product obtained by a reaction between the dialkylaminoacrylate ester of the formula (1) and the alkylhydrazine of the formula (2) is a 1-alkyl-5-hydroxypyrazole represented by the general formula (3). General formula (3)

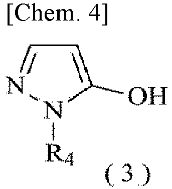

In the formula, R₄ is the same substituent as R₄ of the alkylhydrazine represented by the formula (2), and when the fact that the obtained compound represented by the formula (3) is to be an intermediate raw material of an agricultural chemical or a medicine is taken into account, R₄ is preferably an alkyl group or an aryl group, and these groups may be substituted with halogen, a hydroxy group, an alkoxy group, or a C1 to C6 alkyl group .

Meanwhile, in synthesis of a 1-alkyl-5-hydroxypyrazole by a reaction between a 3-substituted (for example, 3-alkoxy group)-acrylate ester and an alkylhydrazine, the 1-alkyl-5-hydroxypyrazole which is a product is acidic and thus, forms a salt with the alkylhydrazine. Thus, for completing the reaction, it has been necessary to use an excess amount of the alkylhydrazine or to add a basic component that forms a salt with the 1-alkyl-5-hydroxypyrazole.

In contrast, in the production method of the present invention, a 1-alkyl-5-hydroxypyrazole is obtained by reacting the dialkylaminoacrylate ester of the formula (1) and the alkylhydrazine of the formula (2). In this case, a dialkylamine is produced as a byproduct and at the same time, the 1-alkyl-5-hydroxypyrazole which is a product forms a salt with the dialkylamine. Thus, the reaction is completed without addition of an excess amount of a basic component, and thus, a target product can be obtained in a high yield and a high purity in a simple manner.

For producing the 1-alkyl-5-hydroxypyrazole of the present invention, as shown in the reaction formula A, the dialkylaminoacrylate ester of the formula (1) and the alkylhydrazine of the formula (2) are reacted in the absence or presence of a solvent, and a dialkylamine produced as a byproduct is removed, followed by crystallization, whereby a 1-alkyl-5-hydroxypyrazole of the formula (3) which is a target compound can be easily obtained.

In the present invention, the amounts of the dialkylaminoacrylate ester represented by the formula (1) and the alkylhydrazine represented by the formula (2) charged in the reaction are such that the amount of the alkylhydrazine is preferably in the range of 0.5 to 2.0 equivalents relative to 1.0 equivalent of the dialkylaminoacrylate ester, more preferably in the range of 0.8 to 1.2 equivalents, and further preferably 1.0 equivalent. When an excess amount of the dialkylaminoacrylate ester is charged, the yield decreases due to a side reaction between the **1-**alkyl-5-hydroxypyrazole which is the target compound and the dialkylaminoacrylate ester. When an excess amount of the alkylhydrazine is charged, the alkylhydrazine is liable to remain in the crystallization step, and a loss of the 1-alkyl-5-hydroxypyrazole which is the target compound into a mother solution increases, leading to a decreased yield.

In the present invention, the alkylhydrazine represented by the formula (2) can naturally react as it is, that is, as the alkylhydrazine of a high purity as described above, but an alkylhydrazine containing water or other solvents can also react. For example, a commercially available water-containing alkyl (for example, methyl)hydrazine can also be used as it is in the state of the aqueous solution containing water without eliminating water, and can give almost the same result as in the case of using the alkyl(methyl)hydrazine as it is. An alkylhydrazine generally has a low flash point and easily catches fire by oxidation, and the presence of water decreases the flash point of the alkylhydrazine and leads to easy handling, which is preferred.

In the present invention, the reaction can proceed without a solvent, but a solvent may be used to remove the reaction heat. Examples of the solvent include water; alcohols, such as methanol, ethanol, and isopropanol; aliphatic hydrocarbons, such as pentane, hexane, cyclohexane, and petroleum ether; aromatic hydrocarbons, such as toluene and xylene; ethers, such as diethyl ether and diisopropyl ether; halogenated hydrocarbons, such as methylene chloride and chloroform; and nitriles, such as acetonitrile and propionitrile. Among these solvents, alcohols and nitriles are preferred, and alcohols are particularly preferred. One of the solvents may be used alone or two or more thereof may be used in mixture, and the mixing ratio can be any value. The amount of the solvent used is preferably in the range of 0.01 to 5.0 parts by weight relative to 1.0 part by weight of the dialkylaminoacrylate ester represented by the formula (1), and more preferably in the range of 0.1 to 1.0 part by weight.

Regarding the reaction, the dialkylaminoacrylate ester of the formula (1) and the alkylhydrazine of the formula (2) may be simply reacted, and the dialkylaminoacrylate ester of the formula (1) and the alkylhydrazine of the formula (2) may be added to a reaction vessel at once and be mixed and reacted, or the dialkylaminoacrylate ester of the formula (1) and the alkylhydrazine of the formula (2) may be reacted while adding any one compound thereof to the other compound thereof. Since this reaction is accompanied with heat generation, the reaction is preferably carried out while dropwise adding any one of the dialkylaminoacrylate ester represented by the formula (1) and the alkylhydrazine represented by the formula (2) or dropwise adding the dialkylaminoacrylate ester represented by the formula (1) and the alkylhydrazine represented by the formula (2) simultaneously. Since the unreacted dialkylaminoacrylate ester decomposes under a high temperature, it is preferred that the dialkylaminoacrylate ester represented by the formula (1) is dropwise added.

When the reaction is carried out by dropwise adding the alkylhydrazine represented by the formula (2) or by dropwise adding the dialkylaminoacrylate ester represented by the formula (1) and the alkylhydrazine represented by the formula (2) simultaneously, the temperature in dropwise addition of the raw material(s) is preferably a low temperature. The temperature in the dropwise addition of the raw material(s) is preferably in the range of -20⁰C to 60⁰C, and more preferably in the range of 0⁰C to 40⁰C. The reaction temperature after dropwise addition of the raw material(s) is preferably in the range of 0⁰C to 80⁰C, and more preferably in the range of 0⁰C to 60⁰C. When the reaction is carried out by dropwise adding the dialkylaminoacrylate ester represented by the formula (1), the temperature in the dropwise addition of the raw material and the reaction temperature after the dropwise addition of the raw material are preferably in the range of 0⁰C to 80⁰C, and more preferably in the range of 0⁰C to 60⁰C. As the reaction proceeds, the reaction rate decreases, and thus, the reaction temperature is preferably increased in the latter half of the reaction. In the present invention, the time of the dropwise addition of the raw material(s) is preferably in the range of 1 to 20 hours, and more preferably in the range of 2 to 10 hours. The reaction time after the dropwise addition of the raw material(s) is preferably in the range of 1 to 30 hours, and more preferably in the range of 2 to 20 hours.

In the above range of the reaction temperature and in the above range of the reaction time, the positional isomer is produced little, and the final reaction product can be obtained in a high purity and a high yield. When the reaction temperature is higher than the above range and when the reaction time is longer than the above range, a side reaction occurs, leading to a decreased yield and a decreased purity, and thus, the reaction is preferably carried out in the above temperature range and the time range.

The temperature in the dropwise addition of the raw material(s) is preferably lower to avoid an excessive reaction during the dropwise addition. The above ranges of the temperature and time in the dropwise addition do not affect the reaction after the dropwise addition, leading to a preferred result.

In the present invention, a crystal of the 1-alkyl-5-hydroxypyrazole represented by the formula (3) can be easily obtained by crystallization, and it is preferred for the crystallization that a dialkylamine produced as a byproduct in the reaction is removed before the crystallization. The dialkylamine can be removed by an operation of concentration, distillation, neutralization, filtration, or liquid separation. The removal method is not particularly limited and two or more methods may be combined.

When the dialkylamine produced as a byproduct is removed by concentration or distillation, the distillation removal can be achieved at a normal pressure or a reduced pressure. Depending on the type of the dialkylamine produced as a byproduct, the temperature in the concentration or distillation is generally in the range of 30 to 150⁰C, and preferably in the range of 50 to 120⁰C. The degree of pressure reduction may be any in the range of a normal pressure to vacuum.

The dialkylamine produced as a byproduct can be removed by adding an acid to neutralize the dialkylamine to form a dialkylamine salt, and then, removing the produced dialkylamine salt by filtration, washing, or liquid separation. Examples of the acid include mineral acids, such as hydrogen chloride, hydrogen bromide, nitric acid, and sulfuric acid; carboxylic acids, such as formic acid, acetic acid, citric acid, oxalic acid, and benzoic acid; organic sulfonic acids, such as methanesulfonic acid and p-toluenesulfonic acid; an acidic ion exchange resin; and a solid acid, such as zeolite.

In the present invention, the 1-alkyl-5-hydroxypyrazole represented by the formula (3) is obtained as a crystal by recrystallization. It is important to remover the dialkylamine produced as a byproduct before crystallization, and it is preferred to remove 90% or more of the dialkylamine. As a solvent for crystallization, an alcohol, such as methanol, ethanol, or isopropanol; an aliphatic hydrocarbon, such as pentane, hexane, cyclohexane, or petroleum ether; an aromatic hydrocarbon, such as toluene or xylene; an ether, such as diethyl ether or diisopropyl ether; a halogenated hydrocarbon, such as methylene chloride or chloroform; or a nitrile, such as acetonitrile or propionitrile; or an ester, such as ethyl acetate or butyl acetate, is preferred, and acetonitrile or ethyl acetate is particularly preferred.

Through filtration and drying after recrystallization, the 1-alkyl-5-hydroxypyrazole represented by the general formula (3) of a high purity can be obtained. In this case, a simple production method with no need of a purification step by distillation, column chromatography, or the like is provided.

### Examples

The present invention will be described in more detail below with reference to Examples, but the present invention is not to be limited to the examples.

### Example 1

Into a 2 L four-neck flask equipped with a refrigerant cooling reflux tube, 572.8 g (4.0 mol) of ethyl dimethylaminoacrylate and 256.3 g of methanol were added, and while keeping the temperature at 5⁰C or lower, 184.3 g (4.0 mol) of methylhydrazine was added dropwise over 2 hours. After completion of the dropwise addition, the mixture was stirred at 5⁰C for 20 hours. The reaction solution was analyzed by liquid chromatography, and then, the yield of 1-methyl-5-hydroxypyrazole was 95.3%, and the yield of 1-methyl-3-hydroxypyrazole which is a positional isomer was 0.5%.

After completion of the reaction, 253.3 g of the reaction solution was subjected to concentration under a reduced pressure to remove dimethylamine produced as a byproduct together with the solvent. The temperature of the concentrated solution on the concentration was 100⁰C, and the degree of pressure reduction was 5 mmHg. The dimethylamine in the concentrated solution was analyzed and then, was 1.8 g, and thus, 96% of the amount produced as a byproduct was removed. After concentration, 110.0 g of acetonitrile was added, and after dissolution at 60⁰C, the solution was cooled to 0⁰C to precipitate a crystal. The precipitated crystal was taken by filtration, and the collected crystal was dried, thus producing 71.7 g (0.73 mol) of 1-methyl-5-hydroxypyrazole. The yield was 73.1% and the purity was 99.9%. 1-Methyl-3-hydroxypyrazole which is an isomer was not detected.

### Example 2

Into a 300 mL four-neck flask equipped with a refrigerant cooling reflux tube, 143.2 g (1.0 mol) of ethyl dimethylaminoacrylate and 51.3 g of methanol were added, and while keeping the temperature at 5⁰C or lower, 131.6 g (1.0 mol) of 35% methylhydrazine was added dropwise over 2 hours. After completion of the dropwise addition, the mixture was stirred at 5⁰C for 30 hours. The reaction solution was analyzed by liquid chromatography, and then, the yield of 1-methyl-5-hydroxypyrazole was 91.0%, and the yield of 1-methyl-3-hydroxypyrazole which is a positional isomer was 1.5%.

After completion of the reaction, the reaction solution was subjected to concentration under a reduced pressure to remove dimethylamine produced as a byproduct together with the solvent. The temperature of the concentrated solution on the concentration was 100⁰C, and the degree of pressure reduction was 5 mmHg. The dimethylamine in the concentrated solution was analyzed and then, was 2.7 g, and thus, 94% of the amount produced as a byproduct was removed. After concentration, 110.0 g of acetonitrile was added, and after dissolution at 60⁰C, the solution was cooled to 0⁰C to precipitate a crystal. The precipitated crystal was taken by filtration, and the collected crystal was dried, thus producing 60.6 g (0.62 mol) of 1-methyl-5-hydroxypyrazole. The yield was 61.8% and the purity was 99.9%. 1-Methyl-3-hydroxypyrazole which is an isomer was not detected.

### Example 3

Under a degree of pressure reduction of 15 mmHg, 253.3 g of a reaction solution obtained under the same conditions as in Example 1 was concentrated to remove dimethylamine produced as a byproduct together with the solvent. The concentration was terminated at a temperature of the concentrated solution of 100⁰C. The dimethylamine in the concentrated solution was analyzed, and then, was 6.1 g, and 13.5% of the amount produced as a byproduct remained. To the concentrated solution, 110.0 g of acetonitrile was added to dissolve the concentrated solution at 70⁰C. After addition of 35 g of a strong acid ion exchange resin (trade name, AMBERLYST 15DRY, manufactured by ORGANO CORPORATION) and stirring for 10 minutes, the ion exchange resin was separated by thermal filtration. Dimethylamine was not detected in the solution separated by filtration. The solution was cooled to 0⁰C to precipitate a crystal. The precipitated crystal was taken by filtration, and the collected crystal was dried, thus producing 75.8 g (0.77 mol) of 1-methyl-5-hydroxypyrazole. The yield was 77.3% and the purity was 99.9%. 1-Methyl-3-hydroxypyrazole which is an isomer was not detected.

### Example 4

Under a reduced pressure, 253.3 g of a reaction solution obtained under the same conditions as in Example 1 was concentrated to remove dimethylamine produced as a byproduct together with the solvent. The temperature of the concentrated solution on the concentration was 100⁰C, and the degree of pressure reduction was 20 mmHg. The dimethylamine in the concentrated solution was analyzed and then, was 4.7 g, and thus, 10.5% of the amount produced as a byproduct remained (89.5% of the amount produced as a byproduct was removed). To the concentrated solution, 110.0 g of acetonitrile was added, and after dissolution at 60⁰C, the solution was cooled to 0⁰C to precipitate a crystal. The precipitated crystal was taken by filtration, and the collected crystal was dried, thus producing 45.9 g (0.47 mol) of 1-methyl-5-hydroxypyrazole. The yield was 46.8% and the purity was 99.9%. 1-Methyl-3-hydroxypyrazole which is an isomer was not detected.

### Example 5

Into a 100 mL four-neck flask equipped with a refrigerant cooling reflux tube, 11.6 g (0.25 mol) of methylhydrazine and 16.1 g of methanol were added, and while keeping the temperature at 30⁰C, 35.8 g (0.25 mol) of ethyl dimethylaminoacrylate was added dropwise over 2 hours. After completion of the dropwise addition, the mixture was stirred at 60⁰C for 2 hours. The reaction solution was analyzed by liquid chromatography, and then, the yield of 1-methyl-5-hydroxypyrazole was 92.0%, and the yield of 1-methyl-3-hydroxypyrazole which is a positional isomer was 0.3%.

### Example 6

Into a 100 mL four-neck flask equipped with a refrigerant cooling reflux tube, 16.1 g of methanol was added, and while keeping the temperature at 30⁰C, 35.8 g (0.25 mol) of ethyl dimethylaminoacrylate and 11.6 g (0.25 mol) of methylhydrazine were added dropwise over 2 hours. After completion of the dropwise addition, the mixture was stirred at 60⁰C for 2 hours. The reaction solution was analyzed by liquid chromatography, and then, the yield of 1-methyl-5-hydroxypyrazole was 85.0%, and the yield of 1-methyl-3-hydroxypyrazole which is a positional isomer was 0.1%.

### Example 7

Into a 100 mL four-neck flask equipped with a refrigerant cooling reflux tube, 35.8 g (0.25 mol) of ethyl dimethylaminoacrylate and 16.1 g of methanol were added, and while keeping the temperature at 5⁰C or lower, 12.5 g (0.25 mol) of hydrazine hydrate was added dropwise over 2 hours. After completion of the dropwise addition, the mixture was stirred at 30⁰C for 2 hours. The reaction solution was analyzed by liquid chromatography and then, the yield of 5-hydroxypyrazole was 98.5%.

### Comparative Example 1

Into a 300 mL four-neck flask equipped with a refrigerant cooling reflux tube, 116.4 g (1.0 mol) of methyl 3-methoxyacrylate and 64.1 g of methanol were added, and while keeping the temperature at 25⁰C or lower, 46.1 g (1.0 mol) of methylhydrazine was added dropwise over 2 hours. After completion of the dropwise addition, the mixture was stirred at 25⁰C for 20 hours. The reaction solution was analyzed by liquid chromatography, and then, the reaction conversion rates of methyl 3-methoxyacrylate and methylhydrazine were 89.1% and 88.7%, respectively, and the yield of 1-methyl-5-hydroxypyrazole was 77.6%, and the yield of 1-methyl-3-hydroxypyrazole which is a positional isomer was 1.8%.

### Industrial Applicability

The production method of the present invention is useful as an industrial production method because 1-alkyl-5-hydroxypyrazole which is useful as an agricultural herbicide can be obtained from an easily available dialkylaminoacrylate ester in a simple manner and in a high yield and a high purity.

## Claims

1. A method for producing a 1-alkyl-5-hydroxypyrazole represented by the general formula (3), the method comprising subjecting a dialkylaminoacrylate ester represented by the general formula (1) and an alkylhydrazine represented by the general formula (2) to a reaction: General formula (1): 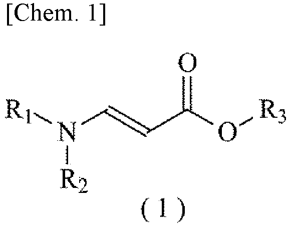
wherein R₁, R₂, and R₃ each independently represent a C1 to C6 alkyl group, 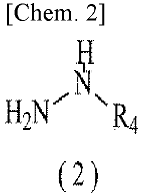
wherein R₄ is a hydrogen atom, a C1 to C6 alkyl group, a C2 to C6 alkenyl group, a C3 to C6 cycloalkyl group, or an aryl group, which groups are optionally substituted with halogen, a hydroxy group, an alkoxy group, or a C1 to C6 alkyl group, 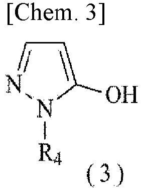
wherein R₄ is a hydrogen atom, a C1 to C6 alkyl group, a C2 to C6 alkenyl group, a C3 to C6 cycloalkyl group, or an aryl group, which groups are optionally substituted with halogen, a hydroxy group, an alkoxy group, or a C1 to C6 alkyl group.

2. The production method according to claim 1, wherein the reaction is carried out by a method in which the alkylhydrazine represented by the general formula (2) or the dialkylaminoacrylate ester represented by the general formula (1) is dropwise added, or a method in which the alkylhydrazine represented by the general formula (2) and the dialkylaminoacrylate ester represented by the general formula (1) are simultaneously dropwise added.

3. The production method according to claim 1 or 2, wherein the alkylhydrazine represented by the general formula (2) is in an aqueous solution.

4. The production method according to any one of claims 1 to 3, wherein a dialkylamine produced as a byproduct is removed and a crystal of the 1-alkyl-5-hydroxypyrazole represented by the general formula (3) is obtained by crystallization.

5. The production method according to claim 4, wherein 90% or more of the dialkylamine produced as a byproduct is removed.

6. The production method according to claim 5, wherein the dialkylamine produced as a byproduct is removed by concentration under a reduced pressure.

7. The production method according to claim 5, wherein the dialkylamine produced as a byproduct is removed by an ion exchange resin.

8. The production method according to any one of claims 1 to 7, wherein R₄ in the general formulae (2) and (3) is a methyl group or an ethyl group.
